(19) Europäisches Patentamt
European Patent Office
Office européen des brevets

(11) Veröffentlichungsnummer: **0 058 895**
**A1**

(12) # EUROPÄISCHE PATENTANMELDUNG

(21) Anmeldenummer: **82101053.5**

(22) Anmeldetag: **12.02.82**

(51) Int. Cl.³: **C 07 D 251/38**
// C07C159/00, C07C125/067

(30) Priorität: **24.02.81 DE 3106724**

(43) Veröffentlichungstag der Anmeldung: **01.09.82**
**Patentblatt 82/35**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Dickoré, Karlfried, Dr.,**
**Nicolai-Hartmann-Strasse 19, D-5090 Leverkusen (DE)**
Erfinder: **Kühle, Engelbert, Dr.,**
**von-Bodelschwingh-Strasse 42,**
**D-5060 Bergisch-Gladbach 2 (DE)**

(54) **Verfahren zur Herstellung von 1-Amino-1,3,5-triazin-2,4(1H, 3H)-dionen.**

(57) Herbizid wirksame 1-Amino-1,3,5-triazin-2,4(1H,3H)-dione der Formel

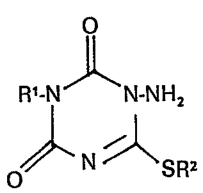

erhält man in guten Ausbeuten, wenn man N-substituierte N-Chlorcarbonyl-carbamidsäure-0-arylester der Formel

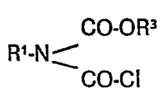

mit Isothiosemicarbazonen der Formel

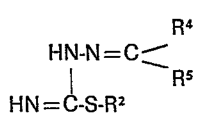

oder deren Hydrohalogeniden bei Temperaturen zwischen 0 und 50°C in Gegenwart eines säurebindenden Mittels umsetzt, anschliessend die hierbei gebildeten offenkettigen Zwischenprodukte (gegebenenfalls ohne Zwischenisolierung) in einer zweiten Stufe auf Temperaturen zwischen 50 und 150°C erhitzt und schliesslich in einer dritten Stufe die hierbei gebildeten 1-Alkylidenamino-1,3,5-triazin-2,4(1H,3H)-dione (gegebenenfalls wiederum ohne Zwischenisolierung) in an sich bekannter Weise in saurem Medium hydrolysiert.

EP 0 058 895 A1

0058895

BAYER AKTIENGESELLSCHAFT 5090 Leverkusen, Bayerwerk

Zentralbereich
Patente, Marken und Lizenzen Bi-by-c
IVa

## Verfahren zur Herstellung von 1-Amino-1,3,5-triazin-2,4(1H, 3H)-dionen

Die vorliegende Erfindung betrifft ein neues Verfahren zur Herstellung von teilweise bekannten 1-Amino-1,3,5-triazin-2,4-(1H, 3H)-dionen, welche als Herbizide verwendet werden können.

Es ist bereits bekannt geworden, daß man 1-Amino-1,3,5-triazin-2,4(1H, 3H)-dione durch Umsetzung von Imidodicarbonsäure-dichloriden mit Hydrohalogeniden von Isothiosemicarbazonen und anschließende säurekatalysierte Hydrolyse der als Zwischenprodukte zunächst gebildeten 1-Alkylidenamino-Derivate herstellen kann (vgl. DE-OS 22 54 200). Dieses Verfahren weist jedoch eine Reihe von Nachteilen auf. So stellt die Verwendung von Imidodicarbonsäure-dichloriden als Ausgangsstoffe einen erheblichen technischen Aufwand dar, da ihre Herstellung nur nach mehrstufigen Verfahren (vgl. DE-OS 23 51 556) oder über schwer zugängliche Ausgangsprodukte (vgl. DE-OS 12 98 095) möglich ist und außerdem die Ausbeuten

nicht befriedigen. Ein weiterer Nachteil des bekannten Verfahrens besteht darin, daß der Ringschluß von Imidodicarbonsäure-dichloriden mit den Hydrohalogeniden von Isothiosemicarbazonen in Gegenwart von drei Molen einer organischen Base in einem organischen Lösungsmittel durchgeführt werden muß. Die technische Durchführung dieses Verfahrens wird durch die erforderliche Rückgewinnung der Lösungsmittel und der Basen zusätzlich erschwert.

Es wurde nun überraschend gefunden, daß man die teilweise bekannten 1-Amino-1,3,5-triazin-2,4(1H, 3H)-dione der allgemeinen Formel

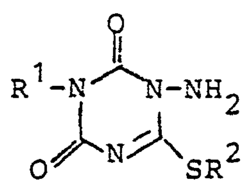 (I),

in welcher

$R^1$ für gesättigte und ungesättigte aliphatische und cycloaliphatische Kohlenwasserstoffreste, araliphatische Kohlenwasserstoff- oder Arylreste, die jeweils durch Halogen, Nitro, Alkyl, Alkoxy, Alkylmercapto, Halogenalkyl, Cyan, Aryl, Aryloxy und/oder Arylmercapto substituiert sein können, oder für heterocyclische Reste steht und

$R^2$ für gesättigte und ungesättigte aliphatische und cycloaliphatische Kohlenwasserstoffreste oder araliphatische Kohlenwasserstoffreste, die jeweils durch

Halogen, Cyan, Nitro, Alkyl, Alkoxy, Alkoxycarbonyl und/oder Alkylmercapto substituiert sein können, steht,

auf technisch einfache Weise in hohen Ausbeuten erhält, wenn man in einer ersten Stufe N-substituierte N-Chlorcarbonyl-carbamidsäure-O-arylester der allgemeinen Formel

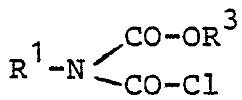

(II),

in welcher

$R^1$ die oben angegebene Bedeutung hat und

$R^3$ für einen Arylrest steht, der jeweils durch Alkyl, Alkoxy, Halogen, Halogenalkyl, Cyan und/oder Nitro substituiert sein kann,

mit einem Isothiosemicarbazon der Formel

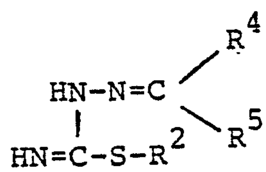

(III),

in welcher

$R^2$ die oben angegebene Bedeutung hat,

$R^4$ für Wasserstoff oder für Alkyl-, Cycloalkyl-, Aralkyl- oder Arylreste, die jeweils durch Halogen, Cyan, Nitro, Alkyl, Alkoxy oder Alkylmercapto substituiert sein können, steht, und

$R^5$ für Alkyl-, Cycloalkyl-, Aralkyl- oder Arylreste, die jeweils durch Halogen, Cyan, Nitro, Alkyl, Alkoxy oder Alkylmercapto substituiert sein können, steht, oder

$R^4$ und $R^5$, zusammen mit dem Alkyliden-C-Atom, für einen 5- bis 7-gliedrigen carbocyclischen Ring stehen,

oder deren Hydrohalogenide bei Temperaturen zwischen 0 und 50°C in Gegenwart eines säurebindenden Mittels und gegebenenfalls in Gegenwart eines Verdünnungsmittels umsetzt, anschließend die hierbei gebildeten offenkettigen Zwischenprodukte der allgemeinen Formel

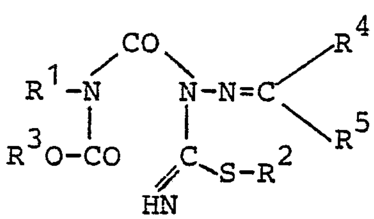

(IVa)

oder

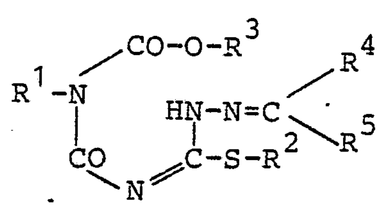

(IVb)

gegebenenfalls ohne Zwischenisolierung, in einer zweiten Stufe gegebenenfalls in Gegenwart eines Verdünnungsmittels auf Temperaturen zwischen 50 und 150°C erhitzt

und schließlich in einer dritten Stufe die hierbei gebildeten 1-Alkylidenamino-1,3,5-triazin-2,4(1 H, 3H)-dione der allgemeinen Formel

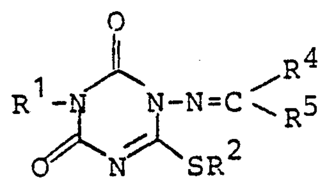

(V),

in welcher

$R^1$, $R^2$, $R^4$ und $R^5$ die oben angegebene Bedeutung haben,

gegebenenfalls ohne Zwischenisolierung, in an sich bekannter Weise in saurem Medium hydrolysiert.

Das erfindungsgemäße Verfahren weist gegenüber dem vorbekannten Verfahren den Vorteil auf, daß nur 1 Mol (bzw. 2 Mol, wenn ein Hydrohalogenid von (III) eingesetzt wird) eines säurebindenden Mittels benötigt wird. Die bei dem erfindungsgemäßen Verfahren als Ausgangsstoffe verwendeten N-substituierten N-Chlorcarbonyl-carbamidsäure-O-arylester sind auf einfache Weise aus technisch leicht zugänglichen Vorprodukten, z.B. durch Phosgenierung von N-substituierten Carbamidsäure-O-arylestern, zugänglich (vgl. DE-AS 1 259 871).

Verwendet man N-Chlorcarbonyl-N-neopentyl-carbamidsäure-O-phenylester und Aceton-S-ethyl-isothiosemicarbazon als Ausgangsstoffe, so kann der Reaktionsablauf durch das folgende Formelschema wiedergegeben werden:

1. Stufe

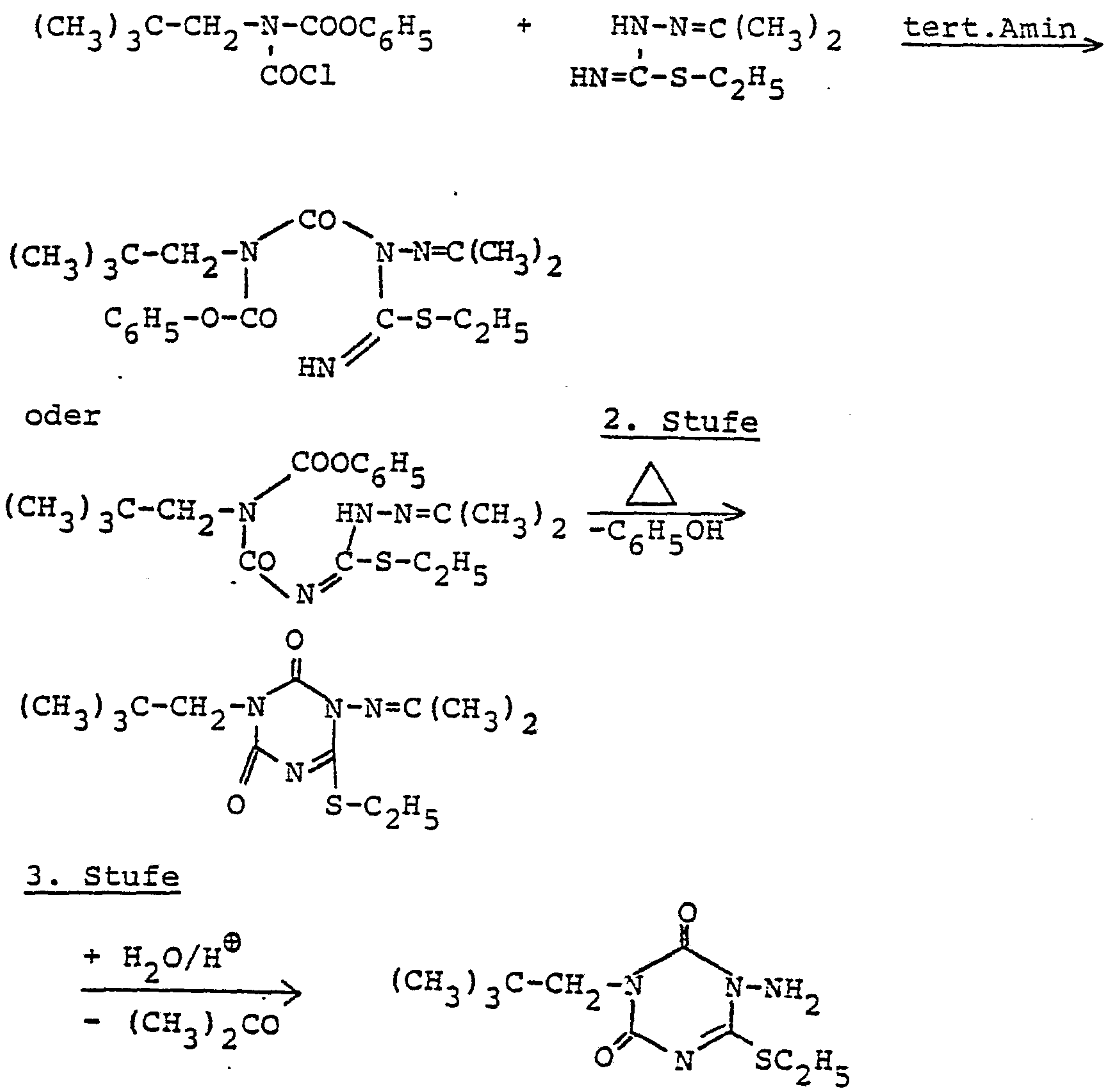


Die als Ausgangsstoffe zu verwendenden N-substituierten N-Chlorcarbonyl-carbamidsäure-O-arylester sind durch die Formel (II) allgemein definiert. In dieser Formel steht $R^1$ vorzugsweise für einen geraden oder verzweigten Alkylrest mit 1-10 C-Atomen, der gegebenenfalls durch Niederalkoxy, Niederalkylmercapto, Halogen (insbesondere Chlor oder Fluor), Cyan und Nitro substituiert sein kann;

ferner für einen Alkenylrest mit 3-8 C-Atomen, einen Alkinylrest mit 3-8 C-Atomen, einen cycloaliphatischen Rest mit 5-8 Ring-C-Atomen, der gegebenenfalls durch Niederalkyl oder Niederalkoxy substituiert sein kann; einen araliphatischen Rest mit 7-12 C-Atomen, wobei das aromatische Ringsystem gegebenenfalls durch Halogen, Nitro, Trihalogenniederalkyl (insbesondere Trifluormethyl), Cyan, Niederalkyl, Niederalkoxy oder Niederalkylmercapto substituiert sein kann; einen aromatischen Rest mit 6-12 C-Atomen, der gegebenenfalls durch Halogen, Nitro, Trihalogen-niederalkyl, (insbesondere Trifluormethyl), Cyan, Niederalkyl, Niederalkoxy oder Niederalkylmercapto substituiert sein kann, oder für einen heterocyclischen Rest mit 5 - 6 Ringatomen, wobei 1 - 3 Heteroatome wie Sauerstoff, Schwefel und/oder Stickstoff im Ringsystem vorhanden sein können.

$R^3$ steht in Formel (II) vorzugsweise für einen Phenylrest, der gegebenenfalls durch Niederalkyl, Niederalkoxy, Halogen, Halogen-niederalkyl (insbesondere Trifluormethyl), Cyan und/oder Nitro substituiert sein kann, oder für einen Naphthylrest.

Die außerdem als Ausgangsstoffe einzusetzenden Isothiosemicarbazone sind durch die Formel (III) allgemein definiert. In dieser Formel steht $R^2$ vorzugsweise für einen geraden oder verzweigten Alkylrest mit 1-6 C-Atomen, der gegebenenfalls durch Niederalkoxy, Niederalkylmercapto, Niederalkoxycarbonyl, Halogen, Cyan oder Nitro substituiert sein kann; einen Alkenylrest mit 3-6 C-Atomen; einen Alkinylrest mit 3-6 C-Atomen; einen cycloaliphatischen Rest mit 5-8 Ring-C-Atomen, der gegebenenfalls durch Niederalkyl oder Niederalkoxy substituiert sein kann; oder für einen araliphatischen Rest mit 7-12 C-Atomen, wobei das aromatische Ringsystem gegebenenfalls durch Niederalkyl, Niederalkoxy, Niederalkylmercapto, Niederalkoxycarbonyl, Halogen, Cyan und/oder Nitro substituiert sein kann.

$R^4$ steht in Formel (III) vorzugsweise für Wasserstoff oder für Alkyl mit 1-3 C-Atomen, Cycloalkyl mit 5-7 C-Atomen, Benzyl oder Arylreste mit 6-10 C-Atomen, wobei diese Reste jeweils durch Halogen, Cyan, Nitro, Niederalkyl, Niederalkoxy oder Niederalkylmercapto substituiert sein können.

$R^5$ steht in Formel (III) vorzugsweise für Alkyl mit 1-3 C-Atomen, Cycloalkyl mit 5-7 C-Atomen, Benzyl oder Aryl mit 6-10 C-Atomen, wobei diese Reste jeweils durch Halogen, Cyan, Nitro, Niederalkyl, Niederalkoxy oder Niederalkylmercapto substituiert sein können.

$R^4$ und $R^5$ können ferner vorzugsweise zusammen mit dem Alkyliden-C-Atom einen 5-7-gliedrigen carbocyclischen Ring bilden. Besonders bevorzugt stehen $R^4$ und $R^5$ für Methyl.

Die Ausdrücke "Niederalkyl", "Niederalkoxy","Niederalkylmercapto", "Halogen-niederalkyl" etc. sollen im Rahmen dieser Erfindung entsprechende Reste mit jeweils 1-4 C-Atomen bedeuten.

Die erfindungsgemäß als Ausgangsstoffe verwendeten N-Chlorcarbonyl-carbamidsäure-O-arylester (II) sind zum Teil bekannt und können durch Umsetzung von N-substituierten Carbamidsäure-arylestern der allgemeinen Formel

$$R^1\text{-NH-CO-OR}^3 \qquad \text{(VI)}$$

in welcher

$R^1$ und $R^3$ die oben angegebene Bedeutung haben,

mit Phosgen in Gegenwart eines tertiären aromatischen Amins (vgl. DE-AS 1 259 871) oder durch Umsetzung von Bis-chlorcarbonylaminen der allgemeinen Formel

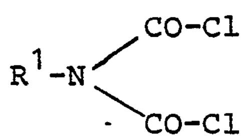

(VII)

in welcher

$R^1$ die oben angegebene Bedeutung hat,

mit Phenolen der allgemeinen Formel

$$\text{HO-R}^3 \qquad \text{(VIII)}$$

in welcher

$R^3$ die oben angegebene Bedeutung hat,

in Gegenwart eines tertiären Amins im Molverhältnis 1:1 (vgl. DE-OS 21 42 496) hergestellt werden. Außerdem erhält man die Ausgangsverbindungen der Formel (II) durch Phosgenierung von N-substituierten Carbamidsäure-arylestern (VI) ohne Zusatz eines säurebindenden Mittels bei Temperaturen von 100-200°C, vorzugsweise bei 130-180°C, gegebenenfalls in Gegenwart eines inerten Lösungsmittels als Verdünnungsmittel; als solche Lösungsmittel kommen beispielsweise Kohlenwasserstoffe, wie z.B. Toluol oder Xylol, oder Chlorkohlenwasserstoffe, wie z.B. Chlorbenzol oder Dichlorbenzol, in Frage. Dieses letztere Verfahren wird vorteilhafterweise dann angewendet, wenn der N-Substituent des Carbamidsäureesters stark verzweigt ist, wie z.B. Isopropyl, Isobutyl oder Neopentyl (vgl. Herstellungsbeispiele).

Die Carbamidsäure-arylester der Formel (VI) sind bereits bekannt oder können nach bekannten Verfahren durch Addition von Isocyanaten an Phenole (vgl. Houben-Weyl, Methoden der organischen Chemie, 4. Aufl. Bd. 8, S. 142 (1952)) oder durch Umsetzung von Kohlensäure-arylesterchloriden mit primären Aminen (vgl. Houben-Weyl, Methoden der organischen Chemie, 4. Aufl. Bd. 8, S. 138 (1952) sowie Herstellungsbeispiele) hergestellt werden. Die Ausgangsverbindungen der Formel (VI) können ferner durch Umsetzung von Kohlensäure-diarylestern mit Aminen hergestellt werden (vgl. Houben-Weyl, Methoden der organischen Chemie, 4. Aufl. Bd. 8, S. 139 (1952)).

Als Beispiele für die erfindungsgemäß verwendbaren N-substituierten N-Chlorcarbonyl-carbamidsäure-O-arylester (II) seien im einzelnen genannt: Die Phenyl-, 2-Chlorphenyl-, 4-Chlorphenyl-, 4-Methylphenyl-, 4-Methoxyphenyl-, 4-Nitrophenyl-, 1-Naphthyl- oder 2-Naphthylester der Methyl-, Ethyl-, 2-Chlorethyl-, 2,2,2-Trifluorethyl-, Propyl-, Isopropyl-, tert.-Butyl-, sek.-Butyl-, Isobutyl-, Pentyl-, Isopentyl- Neopentyl-, 1-Ethylpropyl-, 1,2,2-Trimethylpropyl-, 2-Ethoxymethyl-, 2-Ethylmercaptoethyl-, ω-Cyanohexyl-, Allyl-, Propargyl-, Cyclopropylmethyl-, Cyclopentylmethyl-, Cyclohexylmethyl-, (2,5-Methano-cyclohexyl)-methyl-, Cycloheptylmethyl-, Cyclododekanylmethyl-, Adamantylmethyl-, 2-Furylmethyl-, 2-Pyranylmethyl-, 2-Pyridylmethyl-, 3-Pyridylmethyl-, 4-Pyridylmethyl-, 2-Methylpentyl-, 2-Ethylpentyl-, 2-Methylhexyl-, 2-Ethylhexyl-, Cyclopentyl-, Cyclohexyl-, 2-Methylcyclohexyl-, Benzyl-, 4-Chlorbenzyl-, 4-Nitrobenzyl-, Phenethyl-, Phenyl-, 3-Chlorphenyl-, 4-Chlorphenyl-, 3,5-Dichlorphenyl-, 3,4-Dichlorphenyl-, 3-Trifluormethylphenyl-, 2-Chlor-4-nitro-phenyl-, 3-Methylphenyl-, 4-Methylphenyl-, 3-Methoxy-phenyl-, 1-Naphthyl-, 2-Furyl-, 4-Pyridyl-, 2-Thienyl-, 2-Benzthiazolyl- oder 2-Benzimidazolyl-carbamidsäure.

Die erfindungsgemäß weiterhin als Ausgangsstoffe zu verwendenden Isothiosemicarbazone der Formel (III) bzw. deren Hydrohalogenide sind gleichfalls bekannt oder können nach bekannten Verfahren hergestellt werden, z.B. durch S-Alkylierung von Thiosemicarbazonen (vgl. Houben-Weyl, Methoden der organischen Chemie, 4. Aufl. Band 9, S. 912).

Als Beispiele für solche Isothiosemicarbazone (III) seien im einzelnen genannt:

Aceton-S-methyl-, Aceton-S-ethyl-, Aceton-S-benzyl-, Aceton-S-carbomethoxymethyl-, Isobutyraldehyd-S-methyl-, Benzaldehyd-S-methyl-, Benzaldehyd-S-2-chlorethyl-, Aceton-S-allyl-, Aceton-S-propargyl-, Aceton-S-methoxymethyl-, Aceton-S-cyanmethyl-, Cyclopentanon-S-methyl-, Cyclohexan-on-S-ethyl-, Cyclohexanon-S-carbethoxymethyl-, Cycloheptanon-S-ethyl-, Acetophenon-S-ethyl-, Benzophenon-S-methyl-, Butanon(2)-S-4-chlorbenzyl- oder Butanon(2)-S-ethyl-isothiosemicarbazon, sowie deren Hydrochloride und Hydrobromide.

Die erste und zweite Stufe des erfindungsgemäßen Verfahrens, d.h. die Acylierungsreaktion (II) + (III) ⟶ (IVa) bzw. (IVb) und die Ringschlußreaktion (IVa)/(IVb) ⟶ (V), werden bevorzugt in Gegenwart eines inerten organischen Lösungsmittels als Verdünnungsmittel durchgeführt. In Frage kommen hierfür Kohlenwasserstoffe wie z.B. Toluol, chlorierte Kohlenwasserstoffe wie z.B. Chlorbenzol, oder Alkohole wie z.B. Isopropanol und sec.-Butanol. Zweckmäßigerweise wird für beide Stufen dasselbe Lösungsmittel verwendet.

Die erste Verfahrensstufe (Acylierung) wird unter Zusatz eines säurebindenden Mittels durchgeführt. Als säurebindende Mittel können tertiäre Amine, wie z.B. Triethylamin oder Pyridin, oder anorganische Basen, wie

z.B. Natriumcarbonat oder Natriumhydroxid, verwendet werden. Wenn man die freie Base (III) einsetzt, arbeitet man im Molverhältnis 1:1, verwendet man die Hydrohalogenide von (III), so setzt man das säurebindende Mittel im Molverhältnis 2:1 ein.

Die Reaktionstemperaturen können in einem größeren Bereich variiert werden. Die erste Verfahrensstufe wird im allgemeinen bei 0-50°C, vorzugsweise bei 5-35°C durchgeführt. Die zweite Verfahrensstufe wird im allgemeinen bei Temperaturen zwischen 50 und 150°C, vorzugsweise zwischen 70 und 120°C durchgeführt. Die Anwendung von erhöhtem Druck ist im allgemeinen nicht erforderlich.

Bei der Durchführung des erfindungsgemäßen Verfahrens setzt man auf 1 Mol des N-Chlorcarbonyl-carbamidsäure-O-arylesters der Formel (II) im allgemeinen 0,9-1,1 Mol des Isothiosemicarbazons der Formel (III) bzw. dessen Hydrohalogenids ein; vorzugsweise werden die Komponenten im stöchiometrischen Molverhältnis 1:1 umgesetzt.

Die als Zwischenprodukte gebildeten Verbindungen der Formel (IVa) bzw. (IVb) und die 1-Alkylidenamino-1,3,5-triazin-2,4(1H, 3H)-dione der Formel (V) können, falls gewünscht, jeweils zwischenisoliert werden. Die Aufarbeitung und Isolierung der Zwischenprodukte (V) kann z.B. in der Weise erfolgen, daß man das bei der Cyclisierungsreaktion gebildete, gegebenenfalls substituierte Phenol im Vakuum abdestilliert und den Rückstand durch

Destillation im Hochvakuum oder durch Umlösen, falls erforderlich, reinigt (vgl. Herstellungsbeispiele).

Die anschließende Hydrolyse, zur Abspaltung des als Schutzgruppe dienenden Alkylidenrestes ($=CR^4R^5$), (V) $\rightarrow$ (I), wird in an sich bekannter Weise in saurem Medium durchgeführt (vgl. z.B. DE-OS 22 54 200 bzw. US-PS 4 056 527). Es ist besonders zweckmäßig, die Zwischenprodukte (V) in einem Alkohol wie beispielsweise Isopropanol zu lösen und bei Temperaturen zwischen etwa 40 und 70°C, gegebenenfalls bei vermindertem Druck, mit einer katalytischen Menge einer Säure, beispielsweise einer Mineralsäure wie Schwefelsäure oder einer organischen Sulfonsäure wie p-Toluolsulfonsäure, zu versetzen und die gebildete Carbonylverbindung der Formel $R^4$-CO-$R^5$ zusammen mit einem Teil des als Verdünnungsmittel eingesetzten Alkohols aus dem Reaktionsgemisch abzudestillieren. Die Isolierung der Endprodukte (I) erfolgt in bekannter Weise durch Auskristallisieren und Abfiltrieren; zur weiteren Reinigung können die Endprodukte (I) leicht umkristallisiert werden.

Die erfindungsgemäß herstellbaren 1-Amino-1,3,5-triazin-2,4(1H, 3H)-dione (I) sind großenteils bekannt und besitzen ausgezeichnete herbizide Eigenschaften (vgl. z.B. DE-OS 22 54 200; US-PS 4 056 527; ferner DK-PS 136 067).

Die in der ersten Stufe des erfindungsgemäßen Verfahrens gebildeten, teilweise neuen 1-Alkylidenamino-1,3,5-triazin-2,4(1H, 3H)-dione (V) sind nicht nur als Zwischenprodukte zur Herstellung der entsprechenden 1-Amino-Verbindungen (I) von Interesse, sondern besitzen darüberhinaus auch selbst ausgeprägte herbizide Wirksamkeit (bezüglich der bekannten Verbindungen der allgemeinen Formel (V) vgl. ebenfalls DE-OS 22 54 200; US-PS 4 056 527; DK-PS 136 067).

Die neuen Verbindungen der allgemeinen Formel (V) können in grundsätzlich gleicher Weise wie die vorbekannten Verbindungen formuliert und angewendet werden.

Darüberhinaus ist es möglich, die in 6-Stellung der Verbindungen der allgemeinen Formel (I) und (V) befindlichen $SR^2$-Reste durch Umsetzung mit primären oder sekundären Aminen gegen Alkylamino- bzw. Dialkylaminogruppen auszutauschen, wobei ebenfalls bekannte herbizide Wirkstoffe erhalten werden (vgl. ebenfalls die vorgenannten Druckschriften: DE-OS 22 54 200; US-PS 4 056 527; DK-PS 136 067).

Die nachfolgenden Herstellungs- und Verwendungsbeispiele sollen zur weiteren Erläuterung der Erfindung dienen.

## Herstellungsbeispiele

### A) Endprodukte der Formel (I) und Zwischenprodukte der Formel (V)

Beispiel 1a (3-stufige Verfahrensvariante):

1. Stufe (Acylierung)

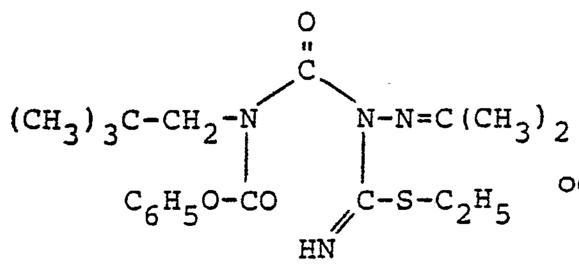

oder

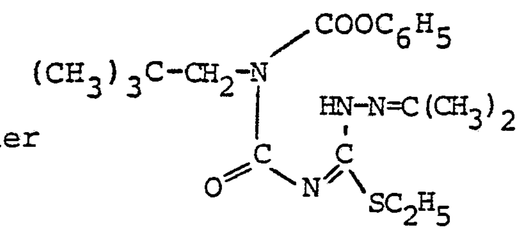

(IVa-1) (IVb-1)

Zu einer Suspension von 48,0 g (0,2 Mol) S-Ethyl-aceton-isothiosemicarbazon-hydrobromid in 250 ml Methylenchlorid gibt man bei 10°C 53,9 g (0,2 Mol) N-Chlorcarbonyl-N-neopentyl-carbamidsäure-O-phenylester und tropft bei 10-15°C 40,4 g (0,4 Mol) Triethylamin zu. Man läßt auf 20°C erwärmen und rührt drei Stunden bei dieser Temperatur, schüttelt mehrmals mit Wasser aus und engt die organische Phase bis zur beginnenden Kristallisation ein. Man saugt bei 0°C ab, wäscht mehrmals mit kaltem Methanol und erhält 48,0 g (61,2 % d.Th.) eines einheitlichen Produktes, (IVa-1) oder (IVb-1), vom Schmelzpunkt 114-116°C.
Eine eindeutige Struktur-Zuordnung für das erhaltene Acylierungsprodukt ist aufgrund des $^{1}$H-NMR-Spektrums nicht möglich.

2. Stufe (Cyclisierung)

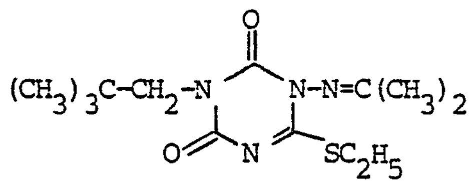

(V-1)

48,0 g (0,122 Mol) des in erster Stufe erhaltenen Acylierungsproduktes (IVa-1) bzw. (IVb-1) erhitzt man 5 Stunden auf 120-150°C, destilliert das gebildete Phenol bei einer Bad-Temperatur von 100°C und einem Druck von 0,1 mbar ab und erhält als Rückstand 34,1 g (93,7 % d.Th.) 1-Isopropylidenamino-6-ethylthio-3-neopentyl-1,3,5-triazin-2,4(1H, 3H)-dion vom Schmelzpunkt 100-102°C und vom Siedepunkt 176°C/0,4 mbar.

3. Stufe (Hydrolyse)

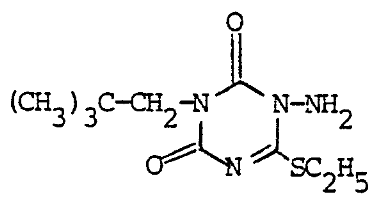

(I-1)

Zu einer Lösung von 34,1 g (0,114 Mol) 1-Isopropylidenamino-6-ethylthio-3-neopentyl-1,3,5-triazin-2,4(1H, 3H)-dion (V-1) in 150 ml Isopropanol gibt man bei 60°C 1,1 g p-Toluolsulfosäure sowie 5,4 ml Wasser und rührt eine Stunde bei 60°C, wobei die Hauptmenge des Reaktionsproduktes auskristallisiert. Man destilliert bei 200-300 mbar ca. 80 ml ab, kühlt auf 0°C, saugt ab und

wäscht mit wenig Methanol. Man erhält 26,8 g (91 % d.Th.) 1-Amino-6-ethylthio-3-neopentyl-1,3,5-triazin-2,4(1H, 3H)-dion vom Schmelzpunkt 202-204°C.

Beispiel 1b (2-stufige Verfahrensvariante)

1. und 2. Stufe

Zu einer Lösung von 31,8 g (0,2 Mol) S-Ethyl-aceton-isothiosemicarbazon und 20,2 g (0,2 Mol) Triethylamin in 200 ml Toluol tropft man bei 15-20°C 53,9 g (0,2 Mol) N-Chlorcarbonyl-N-neopentyl-carbamidsäure-O-phenylester. Man filtriert nach dreistündigem Nachrühren das gebildete Triethylamin-hydrochlorid ab, kocht 3 Stunden unter Rückfluß (110°C), verdampft das Lösungsmittel und destilliert das gebildete Phenol bei einer Bad-Temperatur von 100°C/0,1 mbar ab. Als Rückstand erhält man 51,9 g (87 % d.Th.) 1-Isopropylidenamino-6-ethylthio-3-neopentyl-1,3,5-triazin-2,4-(1H, 3H)-dion (V-1) vom Schmelzpunkt 100-102°C.

3. Stufe

Die Hydrolyse (V-1) $\longrightarrow$ (I-1) erfolgt gemäß Beispiel 1a (3. Stufe).

Analog zu Beispielen 1a und 1b / 1. und 2. Stufe können die folgenden 1-Alkylidenamino-1,3,5-triazin-2,4(1H, 3H)-dione (V) hergestellt werden.

Tabelle 1

| Beispiel Nr. | Strukturformel | Verbindung Nr. | Schmelzpunkt (Siedepunkt) |
|---|---|---|---|
| 2) | 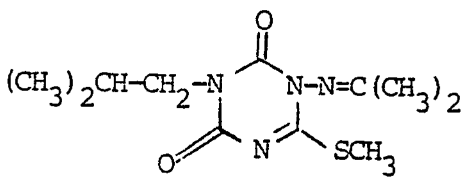 | (V-2) | 165°C/0,38 mbar |
| 3) | 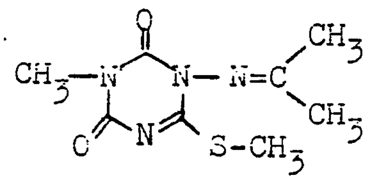 | (V-3) | 130-131°C |
| 4) | 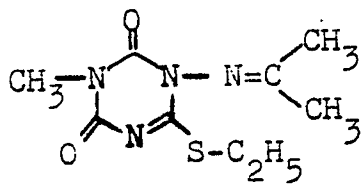 | (V-4) | 121-122°C (173°C/0,5 mbar) |
| 5) | 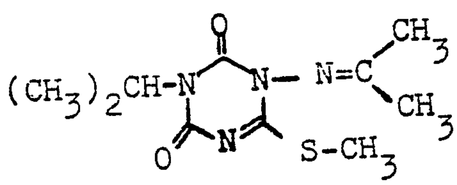 | (V-5) | 110-112°C |
| 6) | 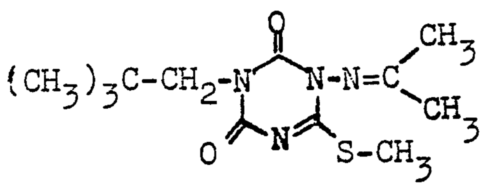 | (V-6) | 122-124°C |
| 7) | 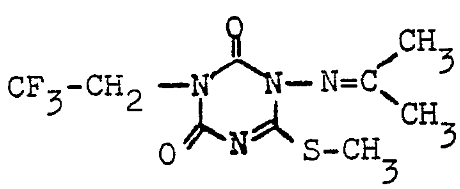 | (V-7) | 142°C (145-150°C/0,3 mbar) |

Tabelle 1 (Fortsetzung)

| Beispiel Nr. | Strukturformel | Verbindung Nr. | Schmelzpunkt (Siedepunkt) |
|---|---|---|---|
| 8) | 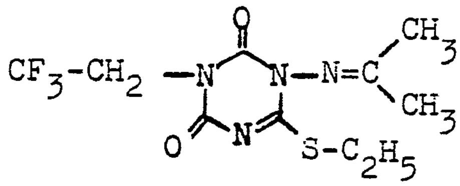 | (V-8) | 112-114°C |
| 9) | 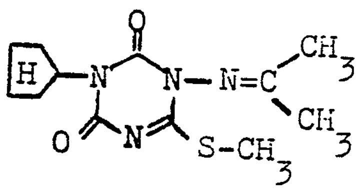 | (V-9) | 107-109°C |
| 10) | 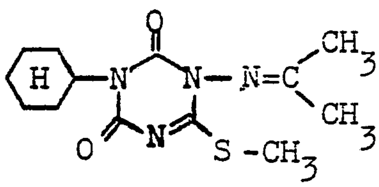 | (V-10) | 111-112°C |

Analog zu Beispielen 1a oder 1b/3. Stufe können die folgenden 1-Amino-1,3,5-triazin-2,4(1H, 3H)-dione (I) hergestellt werden:

Tabelle 2

| Beispiel Nr. | Strukturformel | Verbindung Nr. | Schmelzpunkt |
|---|---|---|---|
| 2) | 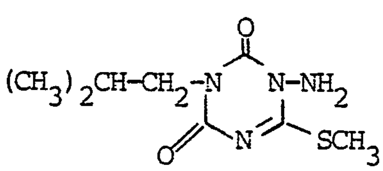 | (I-2) | 167-169°C |

Tabelle 2 (Fortsetzung)

| Beispiel Nr. | Strukturformel | Verbindung Nr. | Schmelzpunkt |
|---|---|---|---|
| 3) | 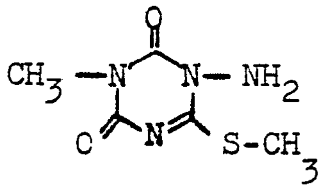 | (I-3) | 174-175°C |
| 4) | 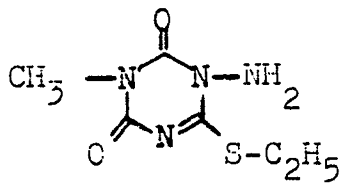 | (I-4) | 133-134°C |
| 5) | 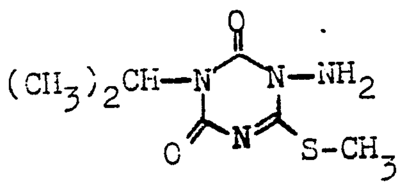 | (I-5) | 148-150°C |
| 6) | 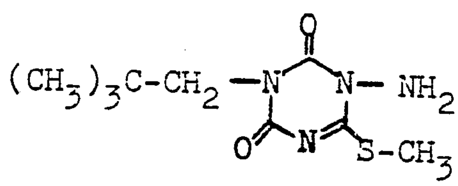 | (I-6) | 229-231°C |
| 7) | 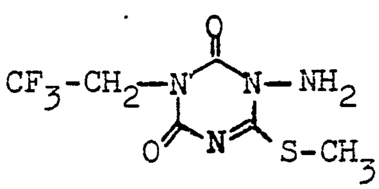 | (I-7) | 147-150°C |

Tabelle 2 (Fortsetzung)

| Beispiel Nr. | Strukturformel | Verbindung Nr. | Schmelzpunkt |
|---|---|---|---|
| 8) | 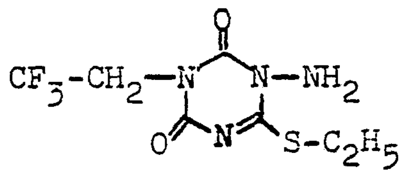 | (I-8) | 135-137°C |
| 9) | 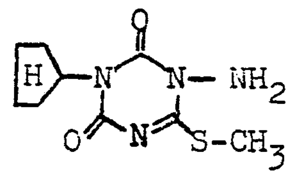 | (I-9) | 158-159°C |
| 10) | 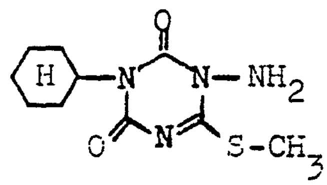 | (I-10) | 177-179°C |

B) Zwischenprodukte der Formeln (VI) und (II)

Beispiel B-1

$$(CH_3)_3C-CH_2-NH-COOC_6H_5 \qquad (VI-1)$$

Der noch nicht bekannte, als Ausgangsverbindung verwendete N-Neopentyl-carbamidsäure-O-phenylester kann beispielsweise, ausgehend von Neopentylamin, folgendermaßen hergestellt werden:

Eine Lösung von 80 g (2 Mol) Natriumhydroxid und 176 g (2 Mol) 99 %iges Neopentylamin in 3,4 Liter Wasser tropft man unter kräftigem Rühren in eine Lösung von 329 g (2,1 Mol) Kohlensäure-phenylesterchlorid in 1 Liter Toluol. Man hält durch Kühlung eine Innen-Temperatur von 10-20°C. Nach beendeter Reaktion trennt man die Phasen, wäscht die organische Phase mit Wasser, filtriert und dampft zur Trockne ein. Man erhält 408 g eines 97 %igen Rohproduktes (95,6 % d.Th.) vom Schmelzpunkt 69-72°C, für weitere Umsetzungen ausreichend rein. Nach dem Umlösen aus 2 Liter Petrolether erhält man 365 g N-Neopentyl-carbamidsäure-O-phenylester (VI-1) vom Schmelzpunkt 77-78°C.

Beispiel B-2

$$(CH_3)_3C-CH_2-\underset{\displaystyle COCl}{N}-COOC_6H_5 \qquad (II-1)$$

Durch eine Lösung von 52 g (0,25 Mol) N-Neopentyl-carbamidsäure-O-phenylester in 250 ml o-Dichlorbenzol wurden bei Siedetemperatur innerhalb von 3 Stunden etwa 150 g Phosgen durchgeleitet. Nach Abdestillieren des Lösungsmittels erhält man durch Destillation 27 g (≙ 40 % Ausbeute) N-Chlorcarbonyl-N-neopentylcarbamidsäure-O-phenylester (II-1) vom Siedepunkt 129-135°C/0,133 mbar.

Patentansprüche

1. Verfahren zur Herstellung von 1-Amino-1,3,5-triazin-2,4(1H, 3H)-dionen der allgemeinen Formel

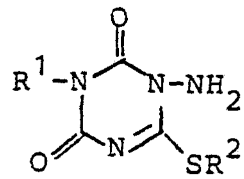

(I)

in welcher

$R^1$ für gesättigte und ungesättigte aliphatische und cycloaliphatische Kohlenwasserstoffreste, araliphatische Kohlenwasserstoff- oder Arylreste, die jeweils durch Halogen, Nitro, Alkyl, Alkoxy, Alkylmercapto, Halogenalkyl, Cyan, Aryl, Aryloxy und/oder Arylmercapto substituiert sein können, oder für heterocyclische Reste steht und

$R^2$ für gesättigte und ungesättigte aliphatische und cycloaliphatische Kohlenwasserstoffreste oder araliphatische Kohlenwasserstoffreste, die jeweils durch Halogen, Cyan, Nitro, Alkyl, Alkoxy, Alkoxycarbonyl und/oder Alkylmercapto substituiert sein können, steht,

dadurch gekennzeichnet, daß man in einer ersten Stufe N-substituierte N-Chlorcarbonylcarbamidsäure-O-arylester der allgemeinen Formel

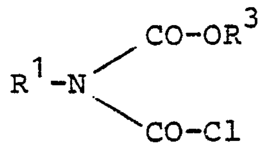 (II),

in welcher

$R^1$ die oben angegebene Bedeutung hat und

$R^3$ für einen Arylrest steht, der jeweils durch Alkyl, Alkoxy, Halogen, Halogenalkyl, Cyan und/oder Nitro substituiert sein kann,

mit einem Isothiosemicarbazon der Formel

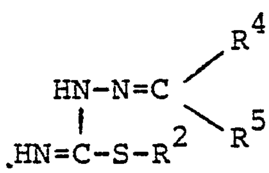 (III)

in welcher

$R^2$ die oben angegebene Bedeutung hat,

$R^4$ für Wasserstoff oder für Alkyl-, Cycloalkyl-, Aralkyl- oder Arylreste, die jeweils durch Halogen, Cyan, Nitro, Alkyl, Alkoxy oder Alkylmercapto substituiert sein können, steht, und

$R^5$ für Alkyl-, Cycloalkyl-, Aralkyl- oder Arylreste, die jeweils durch Halogen, Cyan, Nitro, Alkyl, Alkoxy oder Alkylmercapto substituiert sein können, steht, oder

$R^4$ und $R^5$ zusammen mit dem Alkyliden-C-Atom für einen 5 bis 7-gliedrigen carbocyclischen Ring stehen,

oder deren Hydrohalogenide bei Temperaturen zwischen 0 und 50°C in Gegenwart eines säurebindenden Mittels und gegebenenfalls in Gegenwart eines Verdünnungsmittels umsetzt, anschließend die hierbei gebildeten offenkettigen Zwischenprodukte der allgemeinen Formel

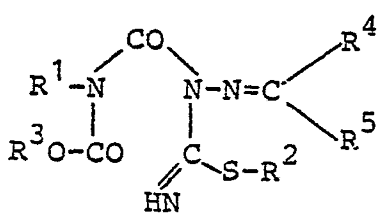

(IVa)

oder

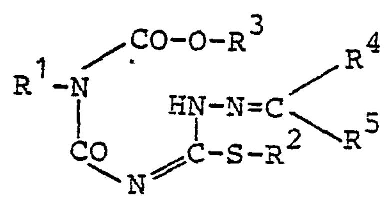

(IVb)

gegebenenfalls ohne Zwischenisolierung, in einer zweiten Stufe gegebenenfalls in Gegenwart eines Verdünnungsmittels auf Temperaturen zwischen 50 und 150°C erhitzt und schließlich in einer dritten Stufe die hierbei gebildeten 1-Alkylidenamino-1,3,5-triazin-2,4(1H, 3H)-dione der allgemeinen Formel

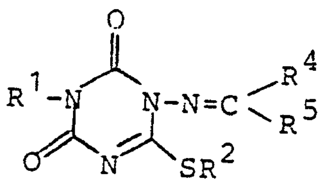

(V) ,

in welcher

$R^1$, $R^2$, $R^4$ und $R^5$ die oben angegebene Bedeutung haben,

gegebenenfalls ohne Zwischenisolierung, in an sich bekannter WEise in saurem Medium hydrolysiert.

2. Verfahren nach Anspruch 1, dadurch gekennzeichnet, daß man die erste Verfahrensstufe bei Temperaturen zwischen 5 und 35°C und die zweite Verfahrensstufe bei Temperaturen zwischen 70 und 120°C durchführt.

3. Verfahren nach Anspruch 1, dadurch gekennzeichnet, daß man auf 1 Mol des N-Chlorcarbonyl-carbamidsäure-O-phenylesters (II) 0,9-1,1 Mol, vorzugsweise 1 Mol, des Isothiosemicarbazons (III) bzw. dessen Hydrohalogenids einsetzt und 1 bzw. 2 Mol eines säurebindenden Mittels verwendet.

4. Verfahren nach Anspruch 1, dadurch gekennzeichnet, daß man als Ausgangsstoffe der Formeln (II) und (III) N-Chlorcarbonyl-N-neopentyl-carbamidsäure-O-phenylester (II-1) und S-Ethyl-aceton-isothiosemicarbazon bzw. dessen Hydrochlorid oder Hydrobromid einsetzt.

0058895

Europäisches Patentamt

# EUROPÄISCHER RECHERCHENBERICHT

Nummer der Anmeldung
EP 82 10 1053

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | DE-A-2 254 200 (BAYER AG) * Seite 5 * | 1 | C 07 D 251/38 // C 07 C 159/00 C 07 C 125/067 |
| A | US-A-3 907 796 (E.I. DU PONT DE NEMOURS AND COMPANY) * Spalten 3,4 * | 1 | |
| D,A | DE-B-1 259 871 (BADISCHE ANILIN- & SODA-FABRIK) * Spalte 3, Zeilen 60-65 * | | |
| E | EP-A-0 034 751 (BAYER AG) * Ansprüche * | 1 | |

RECHERCHIERTE SACHGEBIETE (Int. Cl. 3)

C 07 D 251/00

Der vorliegende Recherchenbericht wurde für alle Patentansprüche erstellt.

| Recherchenort | Abschlußdatum der Recherche | Prüfer |
|---|---|---|
| DEN HAAG | 27-05-1982 | VAN BIJLEN H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument